# EUROPEAN PATENT APPLICATION

(11) **EP 4 623 915 A1**
(43) Date of publication of application: **01.10.2025**
(21) Application number: 23894569.5
(22) Date of filing: 21.11.2023
(51) Int. Cl.: A61K 31/4745, A61K 9/127, A61K 45/00, A61K 47/24, A61K 47/28, A61K 47/34, A61P 35/00, A61P 43/00

(54) **COMBINATION MEDICINE COMPRISING LIPOSOME COMPOSITION HAVING TOPOTECAN OR SALT THEREOF ENCAPSULATED THEREIN AND DNA DAMAGE REPAIR INHIBITOR**

(30) Priority: 21.11.2022 JP 2022185972
(71) Applicant: FUJIFILM Corporation, Tokyo 106-8620 (JP)
(72) Inventor: SHIMOYAMA Susumu, Ashigarakami-gun, Kanagawa 258-8577 (JP); OKADA Ken, Ashigarakami-gun, Kanagawa 258-8577 (JP); MORI Mikinaga, Ashigarakami-gun, Kanagawa 258-8577 (JP)
(74) Representative: Hoffmann Eitle
(86) International application number: PCT/JP2023/041699
(87) International publication number: WO 2024/111564

(57) **Abstract**

An object of the present invention is to provide a medicinal drug obtained by combining a liposome composition encompassing topotecan or a salt thereof and a DNA damage repair inhibitor. According to the present invention, there is provided a medicinal drug including in combination: (A) a liposome composition that contains a liposome having an inner water phase and contains an aqueous solution which is an outer water phase and disperses the liposome, where the liposome encompasses topotecan or a salt thereof, and a lipid constituting the liposome includes dihydrosphingomyelin; and (B) a DNA damage repair inhibitor. The liposome composition and the DNA damage repair inhibitor are administered at the same time or sequentially.

## Description

### Technical Field

The present invention relates to a medicinal drug that is used by combining a liposome composition encompassing topotecan or a salt thereof and a DNA damage repair inhibitor, which are administered at the same time or sequentially.

### Background Art

In chemotherapy, it is often studied that a drug is accumulated at a disease site such as cancer and exposed thereto over a long period of time by means of a liposome composition.

Non-Patent Document 1 describes a combined use of a topoisomerase I inhibitor and a DNA damage repair inhibitor.

Patent Document 1 describes a combined use of topoisomerase I (irinotecan) which is encompassed in a liposome and a PARP inhibitor which is a DNA damage repair inhibitor.

Patent Document 2 describes a liposome composition encompassing topotecan or a salt thereof. Patent Document 3 describes a combined use of a liposome composition encompassing topotecan or a salt thereof and an immune checkpoint inhibitor. Patent Document 4 describes a combined use of a liposome composition encompassing topotecan or a salt thereof and a platinum preparation.

### Prior Art documents

### Patent Documents

Patent Document 1: JP2018-528184A
Patent Document 2: WO2018/181963A
Patent Document 3: WO2019/244979A
Patent Document 4: WO2020/071349A

### Non-Patent Documents

Non-Patent Document 1: CLINICAL CANCER RESEARCH, 2019, Vol. 25, pp. 6581 to 6589: Targeting Topoisomerase I in the Era of Precision Medicine, Anish Thomas et al.

### SUMMARY OF THE INVENTION

The above-described Non-Patent Document 1 describes a combined use of a topoisomerase I inhibitor and a DNA damage repair inhibitor. However, although a strong anti-tumor effect is obtained in a case of combining these substances, strong damage is also given to normal cells as well as hematopoietic stem cells with that proliferate rapidly. Therefore, safety is not sufficient, and further improvement is required.

The above-described Patent Document 1 describes a combined use of a liposome encompassing irinotecan, which is a topoisomerase I inhibitor, and a PARP inhibitor. However, irinotecan is a prodrug (a precursor that is activated by an enzyme in vivo), and it has a variation in drug efficacy. In addition, the anti-tumor effect in a case of being encompassed in a liposome is weak, and side effects such as diarrhea are concerned. Therefore, further improvement is required.

An object of the present invention is to provide a combination of two or more kinds of anti-cancer agents having a high therapeutic effect and few side effects by combining two or more kinds of anti-cancer agents that act according to mechanisms different from each other, in a combined use of a liposome composition encompassing topotecan or a salt thereof and a DNA damage repair inhibitor.

As a result of diligent studies to achieve the above object, the inventors of the present invention found that the above object is achieved by a medicinal drug that is used by combining (A) a liposome composition that contains a liposome having an inner water phase and contains an aqueous solution which is an outer water phase and disperses the liposome, where the liposome encompasses topotecan or a salt thereof, and a lipid constituting the liposome includes dihydrosphingomyelin, and (B) a DNA damage repair inhibitor, which are administered at the same time or sequentially, whereby the present invention has been completed.

That is, the present invention provides the following aspects.
[1] A medicinal drug comprising in combination:
   (A) a liposome composition that contains a liposome having an inner water phase and contains an aqueous solution which is an outer water phase and disperses the liposome, where the liposome encompasses topotecan or a salt thereof, and a lipid constituting the liposome includes dihydrosphingomyelin; and
   (B) a DNA damage repair inhibitor
   in which the liposome composition and the DNA damage repair inhibitor are administered at the same time or sequentially.
[2] The medicinal drug according to [1], in which (A) the liposome composition is administered, and then (B) the DNA damage repair inhibitor is administered.
[3] The medicinal drug according to [1], in which (B) the DNA damage repair inhibitor is administered, and then (A) the liposome composition is administered.
[4] The medicinal drug according to any one of [1] to [3], in which the DNA damage repair inhibitor is at least one selected from a PARP inhibitor, an ATR inhibitor, an ATM inhibitor, a CHK1/2 inhibitor, a WEE1 inhibitor, a DNA-PK inhibitor, or inhibitors that inhibit pathways in which these inhibitors are involved.
[5] The medicinal drug according to any one of [1] to [4], in which the lipid constituting the liposome further includes a lipid modified with cholesterol and polyethylene glycol.
[6] The medicinal drug according to any one of [1] to [5], in which a formulation ratio of a lipid modified with polyethylene glycol to an entire lipid constituting the liposome is 2% by mole to 10% by mole.
[7] The medicinal drug according to any one of [1] to [6], in which a lipid modified with polyethylene glycol is diacylphosphatidylethanolamine modified with polyethylene glycol or methoxypolyethylene glycol.
[8] The medicinal drug according to any one of [1] to [7], in which a formulation ratio of cholesterol to an entire lipid constituting the liposome is 35% to 43% by mole.
[9] The medicinal drug according to any one of [1] to [8], in which the inner water phase of the liposome contains an ammonium salt.
[10] The medicinal drug according to any one of [1] to [9], in which the DNA damage repair inhibitor is a PARP inhibitor.
[11] The medicinal drug according to any one of [1] to [10], in which a dose per administration of the topotecan or salt thereof encompassed in (A) the liposome composition is such that a dose rate is 0.1 mg/m² body surface area to 10 mg/m² body surface area in terms of topotecan.
[12] The medicinal drug according to any one of [1] to [11], in which (A) the liposome composition is administered once every 1 week to 8 weeks.
[13] A treatment method for a disease (preferably cancer) of a subject, where the treatment method is used by combining (A) a liposome composition that contains a liposome having an inner water phase and contains an aqueous solution which is an outer water phase and disperses the liposome, where the liposome encompasses topotecan or a salt thereof, and a lipid constituting the liposome includes dihydrosphingomyelin, and (B) a DNA damage repair inhibitor, which are administered at the same time or sequentially at an effective dose and in an administration period, where a therapeutically synergistic action is exhibited in a subject.
[14] A maintenance therapy for a disease (preferably cancer) of a subject, the maintenance therapy being used by combining (A) a liposome composition that contains a liposome having an inner water phase and contains an aqueous solution which is an outer water phase and disperses the liposome, where the liposome encompasses topotecan or a salt thereof, and a lipid constituting the liposome includes dihydrosphingomyelin, and (B) a DNA damage repair inhibitor, which are administered at the same time or sequentially at an effective dose and in an administration period, which are required to prevent recurrence of a disease of a subject.
[15] A medicinal drug for use in treatment of a disease (preferably cancer) of a subject, the medicinal drug comprising in combination:
   (A) a liposome composition that contains a liposome having an inner water phase and contains an aqueous solution which is an outer water phase and disperses the liposome, where the liposome encompasses topotecan or a salt thereof, and a lipid constituting the liposome includes dihydrosphingomyelin; and
   (B) a DNA damage repair inhibitor
   in which the liposome composition and the DNA damage repair inhibitor are administered at the same time or sequentially.
[16] Use of (A) a liposome composition that contains a liposome having an inner water phase and contains an aqueous solution which is an outer water phase and disperses the liposome, the liposome composition being such that the liposome contains topotecan or a salt thereof, and a lipid constituting the liposome includes dihydrosphingomyelin, and (B) a DNA damage repair inhibitor,
   in which the use is for producing a medicinal drug including in combination;
   (A) a liposome composition that contains a liposome having an inner water phase and contains an aqueous solution which is an outer water phase and disperses the liposome, where the liposome encompasses topotecan or a salt thereof, and a lipid constituting the liposome includes dihydrosphingomyelin, and
   (B) a DNA damage repair inhibitor
   in which the liposome composition and the DNA damage repair inhibitor are administered at the same time or sequentially.

### Effect of the Invention

The medicinal drug according to the aspect of the present invention is used by combining a liposome composition encompassing topotecan or a salt thereof and a DNA damage repair inhibitor, which are administered at the same time or sequentially, and thus it is possible to reduce the enhancement of side effects on normal tissues (normal organs, bone marrow, and the like) and to exhibit a strong drug efficacy with respect to cancer. Therefore, it is possible to provide a medicinal drug that enhances at least one effect of curing of cancer, prevention of cancer, or suppression of recurrence of cancer.

In addition, since the medicinal drug according to the aspect of the present invention has a remarkable effect of suppressing cell proliferation in cancer tissues even at a low dose, it is possible to achieve a desirable treatment that is not only highly safe for a subject including a patient but also has a low physical burden and high convenience.

### BRIEF DESCRIPTION OF THE DRAWINGS

Fig. 1 shows a change in tumor volume as data related to drug efficacy based on the administration day in Test Example 1.
Fig. 2 shows a change in body weight as data on safety based on the administration day in Test Example 1.
Fig. 3 shows a change in tumor volume as data related to drug efficacy based on the administration day in Test Example 2.
Fig. 4 shows a change in body weight as data on safety based on the administration day in Test Example 2.
Fig. 5 shows the results related to the combined use of ceralasertib in Capan-1 (pancreatic cancer cell line) in the isobologram analysis in Test Example 3.
Fig. 6 shows the results related to the combined use of M4076 in Capan-1 (pancreatic cancer cell line) in the isobologram analysis in Test Example 3.
Fig. 7 shows the results related to the combined use of M3814 in Capan-1 (pancreatic cancer cell line) in the isobologram analysis in Test Example 3.
Fig. 8 shows the results related to the combined use of Zn-C3 in Capan-1 (pancreatic cancer cell line) in the isobologram analysis in Test Example 3.
Fig. 9 shows the results related to the combined use of olaparib in Capan-1 (pancreatic cancer cell line) in the isobologram analysis in Test Example 3.
Fig. 10 shows the results related to the combined use of ceralasertib in ES-2 (ovarian cancer cell line) in the isobologram analysis in Test Example 3.
Fig. 11 shows the results related to the combined use of M4076 in ES-2 (ovarian cancer cell line) in the isobologram analysis in Test Example 3.
Fig. 12 shows the results related to the combined use of prexasertib in ES-2 (ovarian cancer cell line) in the isobologram analysis in Test Example 3.
Fig. 13 shows the results related to the combined use of M3814 in ES-2 (ovarian cancer cell line) in the isobologram analysis in Test Example 3.
Fig. 14 shows the results related to the combined use of Zn-C3 in ES-2 (ovarian cancer cell line) in the isobologram analysis in Test Example 3.
Fig. 15 shows the results related to the combined use of olaparib in ES-2 (ovarian cancer cell line) in the isobologram analysis in Test Example 3.
Fig. 16 shows the results related to the combined use of ceralasertib in DMS 114 (lung cancer cell line) in the isobologram analysis in Test Example 3.
Fig. 17 shows the results related to the combined use of M4076 in DMS 114 (lung cancer cell line) in the isobologram analysis in Test Example 3.
Fig. 18 shows the results related to the combined use of prexasertib in DMS 114 (lung cancer cell line) in the isobologram analysis in Test Example 3.
Fig. 19 shows the results related to the combined use of M3814 in DMS 114 (lung cancer cell line) in the isobologram analysis in Test Example 3.
Fig. 20 shows the results related to the combined use of Zn-C3 in DMS 114 (lung cancer cell line) in the isobologram analysis in Test Example 3.
Fig. 21 shows the results related to the combined use of olaparib in DMS 114 (lung cancer cell line) in the isobologram analysis in Test Example 3.

### THE EMBODIMENTS FOR CARRYING OUT THE INVENTION

In the present invention, the range represented by "to" includes the values at both ends thereof unless otherwise specified.

The subject includes humans and mammals other than humans. Examples of mammals other than humans include monkeys, dogs, cats, cows, horses, mice, and rats.

The treatment may be any treatment or therapy that achieves a desired therapeutic effect, for example, inhibition or delay of progression of a condition, and includes slowing down a rate of progression, pausing the rate of progression, improving the condition, healing or remitting the condition (whether partial or complete), preventing, delaying, reducing, or arresting one or a plurality of symptoms and/or signs of the condition, and prolonging a subject or subject's survival over that expected in the absence of treatment.

The treatment also includes prevention. For example, treating a subject who is susceptible to or at risk of onset or recurrence of cancer may prevent or delay the onset or recurrence of cancer in the subject.

The treatment may include inhibition of cancer growth including complete remission of cancer, and/or inhibition of cancer metastasis. The cancer growth refers to the transformation of cancer into a more developed form. Examples of an indicator for measuring the inhibition of cancer growth include decreased survival of cancer cells, decreased tumor volume or morphology (for example, determined using computed tomography (CT), ultrasonography, or other diagnostic imaging methods), delayed tumor growth, destruction of tumor vasculature, improved scores of delayed hypersensitivity skin test, increased activity of cytolytic T-lymphocytes, and decreased levels of tumor-specific antigens.

In the present invention, tumor, malignant tumor, cancer, malignant neoplasm, carcinoma, sarcoma, and the like are collectively referred to as "tumor" or "cancer". In addition, the term "tumor" or "cancer" includes those that have recurred after the treatment of cancer. The term "tumor" includes all malignant or benign neoplastic cell growth and proliferation, as well as pre-cancerous and cancerous cells and tissues.

The term "effective amount" is a dose required to achieve a desired therapeutic or prophylactic result, including the period and amount of administration. The "effective amount" of the medicinal drug according to the embodiment of the present invention may vary depending on the disease state, age, sex, and body weight of a subject (or individual), the ability of the medicinal drug to elicit a desired response in the subject (or individual).

The term "co-administration" refers to administering a first therapy and a second therapy in a combination therapy at a time interval of about 15 minutes or less, such as any of about 10 minutes, about 5 minutes, or about 1 minute or less. In a case where the first therapy and the second therapy are administered simultaneously, the first therapy and the second therapy can be contained in the same composition (for example, a composition that includes both the first therapy and the second therapy), or can be contained in separate compositions (for example, the first therapy is contained in one composition and the second therapy is contained in another composition).

The term "sequential administration" refers to administering a first therapy and a second therapy in a combination therapy at a time interval of more than about 15 minutes, such as any of about 20 minutes, about 30 minutes, about 40 minutes, about 50 minutes, about 60 minutes or longer (1 day, 2 days, 3 days, 1 week, 2 weeks, 3 weeks, 4 weeks, 8 weeks, 12 weeks, 16 weeks, or the like). In the present invention, the sequential administration also includes the first administration of the first therapy and the first administration of the second therapy. In addition, in the present invention, the sequential administration also includes the administration of the second therapy after the administration of the first therapy (after a predetermined time (for example, after 1 week)). The first therapy and the second therapy may be contained in separate compositions, which may be contained in the same package or kit or may be contained in different packages or kits.

The term "maintenance therapy" is a treatment method in which, after obtaining a certain effect by surgery or drug therapy for cancer, a treatment using the same or different drugs is continued as much as possible for the intended purpose of preventing recurrence or progression of cancer.

The term "retention in the blood" means a property in which a drug in a state of being encapsulated in a liposome is present in the blood in a subject to which a liposome composition has been administered.

The term "average particle diameter of liposome" means an average particle diameter (preferably a cumulant average particle diameter) measured using a dynamic light scattering method unless otherwise specified. Examples of commercially available determination devices using dynamic light scattering include a concentrated system particle size analyzer FPAR-1000 (manufactured by Otsuka Electronics Co., Ltd.), a Nanotrac UPA (manufactured by Nikkiso Co., Ltd.), and a Nanosizer (manufactured by Malvern Panalytical Ltd.). It is also possible to calculate a volume average particle diameter and a number average particle diameter of the liposome by the conversion equation specific to the determination device of each manufacturer. In order to measure particles in the vicinity of 100 nm, the distribution of particles cannot be accurately captured by a static light scattering method or the like, and measurement by the dynamic light scattering method is preferable.

Hereinafter, the present invention will be described in detail.

The present invention provides a medicinal drug comprising, in combination, (A) a liposome composition that encompasses a liposome having an inner water phase and encompasses an aqueous solution which is an outer water phase and disperses the liposome, where the liposome encompasses topotecan or a salt thereof, and a lipid constituting the liposome includes dihydrosphingomyelin; and (B) a DNA damage repair inhibitor, in which the liposome composition and the DNA damage repair inhibitor are administered at the same time or sequentially.

The above-described WO2018/181963A describes a liposome composition encompassing topotecan or a salt thereof; however, it does not describe a combination of the liposome composition and a DNA damage repair inhibitor. Therefore, the medicinal drug according to the embodiment of the present invention cannot be easily conceived from the description in WO2018/181963A.

The above-described WO2019/244979A describes a combined use of a liposome composition encompassing topotecan or a salt thereof and an immune checkpoint inhibitor. The population such as the number or the kind of immune cells in the tumor environment is changed by the action of the liposome encompassing topotecan or a salt thereof. T cells or antigen-presenting cells, on which an immune checkpoint inhibitor acts, enhance an anti-tumor effect against immune cells in the tumor environment, whereby an anti-tumor effect is exhibited. It is a technique that has an anti-tumor effect due to an indirect synergistic effect through T cells or antigen-presenting cells.

On the other hand, a combination of a liposome composition encompassing topotecan or a salt thereof and a DNA damage repair inhibitor has a direct synergistic effect of damage to a gene (DNA) of a cancer cell and inhibition of repair of the damage thereto. Therefore, the medicinal drug according to the embodiment of the present invention cannot be easily conceived from the description in WO2019/244979A.

The above-described WO2020/071349A describes a combined use of a liposome composition encompassing topotecan or a salt thereof and a platinum preparation. A liposome composition encompassing topotecan or a salt thereof and a platinum preparation causes together damage to a gene (DNA) of a cancer cell, whereby an anti-tumor effect is obtained according to a similar mechanism. Therefore, the medicinal drug according to the embodiment of the present invention cannot be easily conceived from the description in WO2020/071349A.

In addition, in the cancer (tumor) resistant to DNA damage, the effect of the combination of the liposome composition encompassing topotecan or a salt thereof and the platinum preparation may be weakened.

### (Liposome)

A liposome is a closed small vesicle formed of a lipid bilayer membrane that is formed from a lipid, and an aqueous phase (an inner water phase) is included in the space of the closed small vesicle. The inner water phase contains water. The liposome is generally present in a state of being dispersed in an aqueous solution (an outer water phase) outside the closed vesicle. The liposome may be a single lamella (which is also called a single-layer lamella or uni-lamella, where the bilayer membrane has a single layered structure) or may be a multi-layer lamella (which is also called a multi-lamella and has a structure of a large number of bilayer membranes, having an onion-like shape, where the individual layers are separated by an aqueous layer). However, in the present invention, a single lamellar liposome is preferable from the viewpoint of safety and stability in use applications to a medicinal drug.

The form of the liposome is not particularly limited as long as the liposome is a liposome capable of encompassing a drug. The term "encompassing" means taking a form in which a drug is encompassed in the inner water phase of the liposome. Examples thereof include a form in which a drug is enclosed in a closed space formed from a membrane and a form in which a drug is encompassed in the membrane itself, where a combination of these may be good.

In general, the average particle diameter of the liposome is 10 nm to 1,000 nm, preferably 20 nm to 500 nm, more preferably 30 nm to 300 nm, still more preferably 30 nm to 200 nm, even still more preferably 30 nm to 150 nm, and particularly preferably 50 nm to 150 nm. The liposome preferably has a spherical shape or a shape close thereto.

In a case of expecting the enhanced permeability and retention effect (the EPR effect), the diameter is preferably substantially 50 to 200 nm, the diameter is more preferably substantially 50 to 150 nm, and the diameter is still more preferably substantially 50 to 100 nm. The term "substantially" means that at least 75% of the number of liposomes is within the specified diameter range. The above-described "at least 75%" is more preferably at least 80% and still more preferably at least 90%.

It is noted that in the present invention, the term "average particle diameter" means an average particle diameter (preferably, a cumulant average particle diameter) measured by using a dynamic light scattering method unless otherwise specified. The "average particle diameter" can be measured by using a device that can measure the average particle diameter according to a light scattering method.

The component constituting the lipid bilayer of the liposome is selected from lipids. It is preferable that the liposome in the present invention includes a hydrophilic polymer-modified diacylphosphatidylethanolamine, dihydrosphingomyelin, and cholesterol as the constitutional components of the liposome membrane.

The liposome according to the embodiment of the present invention contains dihydrosphingomyelin. The retention of the liposome in the blood can be improved by using dihydrosphingomyelin. In addition, it is possible to improve the partition wall properties of the liposome membrane and prevent the leakage of topotecan or a salt thereof.

Dihydrosphingomyelin generally has two long-chain alkyl groups in the molecule, and examples thereof include dihydrosphingomyelin having two long-chain alkyl groups respectively having 16 carbon atoms, dihydrosphingomyelin having a long-chain alkyl group having 16 carbon atoms and a long-chain alkyl group having 18 carbon atoms, and dihydrosphingomyelin having a long-chain alkyl group having 16 carbon atoms and a long-chain alkyl group having 20 to 24 carbon atoms.

From the viewpoint of preventing leakage of a drug from the liposome, it is preferable to use, as the dihydrosphingomyelin, the following compound having a long-chain alkyl group having 16 carbon atoms and a long-chain alkyl group having 18 carbon atoms. This is because the melting point becomes higher as the number of carbon atoms is larger, and therefore a liposome membrane having high partition wall properties can be formed.

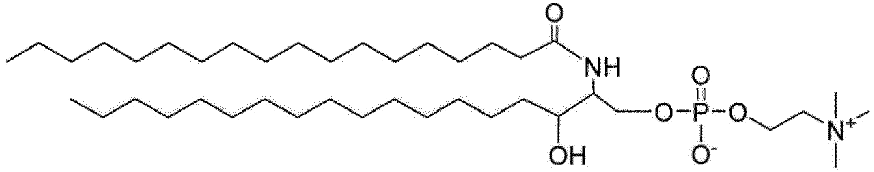

As the dihydrosphingomyelin, for example, dihydrosphingomyelin obtained by reducing sphingomyelin derived from natural products by a general method may be used, or dihydrosphingomyelin obtained by synthesis may be used.

Since most dihydrosphingomyelins derived from natural products such as chicken eggs generally have two long-chain alkyl groups respectively having 16 carbon atoms, it is preferable to use dihydrosphingomyelin obtained by chemical synthesis, from the viewpoint that dihydrosphingomyelin having a long-chain alkyl group having 16 carbon atoms and a long-chain alkyl group having 18 carbon atoms can be obtained with high purity.

The ratio of dihydrosphingomyelin in the total lipids constituting the liposome is preferably 30% to 80% by mole, more preferably 40% to 70% by mole, and still more preferably 50% to 60% by mole.

It is preferable that the liposome according to the embodiment of the present invention contains a lipid modified with polyethylene glycol (hereinafter, referred to as a PEG-modified lipid). It is noted that as the polyethylene glycol, a derivative thereof can also be used, and examples the derivative thereof include methoxypolyethylene glycol. Hereinafter, in a case of being referred to as PEG modification, the PEG modification also applies to a derivative of polyethylene glycol.

Examples of the PEG-modified lipid include a PEG-modified phospholipid, a PEG-modified monoglyceride, a PEG-modified diglyceride, a PEG-modified sorbitan fatty acid ester, a PEG-modified monoalkyl ether, and a PEG-modified sterol. The above-described hydrophilic polymers may be each used alone or in a combination of two or more kinds thereof.

The molecular weight of the polyethylene glycol is not particularly limited and is 500 to 10,000 daltons, preferably 1,000 to 7,000 daltons, and more preferably 2,000 to 5,000 daltons.

Examples of the PEG-modified lipid include 1,2-distearoyl-3-phosphatidylethanolamine-polyethylene glycol such as 1,2-distearoyl-3-phosphatidylethanolamine-PEG2000 (manufactured by NOF Corporation), distearoyl glycerol PEG2000 (manufactured by NOF Corporation), or 1,2-distearoyl-3-phosphatidylethanolamine-PEG5000 (manufactured by NOF Corporation), and cholesterol-polyethylene glycol such as cholesterol-PEG600 (manufactured by Merck KGaA).

From the viewpoint of the general-purpose properties and the retention in the blood, the PEG-modified lipid is preferably a PEG-modified phospholipid or a PEG-modified monoalkyl ether and is more preferably a PEG-modified phospholipid.

Among the PEG-modified phospholipids, PEG-modified phosphatidylethanolamine is preferable, and diacylphosphatidylethanolamine modified with polyethylene glycol or methoxypolyethylene glycol is more preferable.

The ratio of the PEG-modified lipid in the total lipids constituting the liposome is preferably 1% to 15% by mole and more preferably 2% to 10% by mole.

It is preferable that the liposome according to the embodiment of the present invention contains cholesterol. The addition of cholesterol to the liposome is expected to lower the fluidity of the membrane of the liposome, for example, by filling the gaps in the membrane of the liposome.

The ratio of cholesterol in the lipid constituting the liposome is preferably 20% by mole to 50% by mole, more preferably 30% by mole to 45% by mole, and still more preferably 35% by mole to 43% by mole.

### (Topotecan or salt thereof)

The liposome according to the embodiment of the present invention encompasses topotecan or a salt thereof. The topotecan has a chemical name of (10-[(dimethylamino)methyl]-4-ethyl-4,9-dihydroxy-1H-pyrano[3',4':6,7]indolidino[1,2-b]quinoline-3,14(4H,12H)dione and is an anti-cancer agent having an inhibitory action on topoisomerase activity. In the present invention, the topotecan may be topotecan itself or may be a salt thereof, which is acceptable as a pharmaceutical drug, or it may be a prodrug that liberates topotecan in vivo. In the present invention, it is preferable to use topotecan hydrochloride.

The concentration of the topotecan or salt thereof in the liposome can be measured, for example, by liquid chromatography/ultraviolet-visible absorbance detection. In addition, the sulfate ions concentration in the inner water phase of the liposome can be measured, for example, by ion chromatography.

The content of the topotecan or salt thereof in the liposome composition is not particularly limited; however, it is preferably 0.025 to 20 mg/mL and more preferably 0.25 to 10 mg/mL with respect to the liposome composition.

The amount of the topotecan or salt thereof encompassed in the liposome with respect to the lipid that forms the liposome membrane is preferably 0.1 to 1.5 and more preferably 0.2 to 0.3 in terms of a molar ratio, from the viewpoint of the release rate from the liposome, the osmotic pressure inside the liposome, and the shape of the liposome due to the precipitated drug.

In a case where the molar ratio of the topotecan or a salt thereof to the lipid is too low, the area of the liposome membrane with respect to the unit drug amount is increased, the release rate of the drug from the liposome is increased, and therefore the function of improving the retention in the blood is impaired. On the other hand, in a case where the molar ratio of the topotecan or a salt thereof to the lipid is too high, the osmotic pressure inside the liposome is increased with an increased amount of the drug dissolved, which results in the destruction of the liposome, or in a case where the drug is precipitated inside the liposome, the precipitated solid grows large, which results in the deformation of the liposome shape.

### (Inner water phase)

The liposome composition according to the embodiment of the present invention contains a liposome having an inner water phase, and an aqueous solution which is an outer water phase and disperses the liposome, where the liposome encompasses topotecan or a salt thereof. The inner water phase of the liposome preferably contains an ammonium salt. Examples of the ammonium salt include an ammonium sulfate salt, ammonium citrate, ammonium phosphate, ammonium tartrate, ammonium succinate, ammonium fatty acid, ammonium chloride, and sucrose octasulfate as ammonium sulfate. Among them, since the retention stability of a drug can be improved by reducing the solubility of the drug in the liposome to precipitate the drug, an ammonium sulfate salt, an ammonium citrate salt, an ammonium phosphate salt, or a sucrose octasulfate as ammonium sulfate is preferable, and an ammonium sulfate salt is particularly preferable.

In a case where the inner water phase according to the embodiment of the present invention contains ammonium sulfate, the molar ratio of sulfate ions in the inner water phase to the total molar sum of topotecan in the topotecan or salt thereof encompassed in the liposome composition according to the embodiment of the present invention is preferably 0.36 or more, more preferably 0.4 or more, still more preferably 0.4 or more and 1.8 or less, and particularly preferably 0.6 or more and 1.8 or less. In a case of setting the molar ratio of sulfate ions as described above, it is possible to suppress leakage of the topotecan or a salt thereof from the liposome in the blood.

In addition, in a case where the inner water phase according to the embodiment of the present invention contains ammonium sulfate, the ratio of sulfate ions contained in ammonium sulfate to sulfate ions of the entire anti-tumor agent (the inner water phase ratio of the sulfate ions) is preferably at least 80% and more preferably 90% or more. At the same time, the ratio of the topotecan or salt thereof contained in the inner water phase of the liposome to the topotecan or salt thereof of the entire anti-tumor agent (the inner water phase ratio of the drug) is preferably at least 80% and more preferably 90% or more.

### (Outer water phase)

The liposome composition according to the embodiment of the present invention contains a liposome having an inner water phase, and an aqueous solution which is an outer water phase and disperses the liposome, where the liposome encompasses topotecan or a salt thereof. The pH of the outer water phase of the liposome composition according to the embodiment of the present invention is preferably neutral, and specifically, it is preferably a pH of about 5.5 to 8.5.

### (Method for producing liposome composition)

A production method for the liposome according to the embodiment of the present invention is not particularly limited; however, the production method can be carried out with reference to, for example, WO2018-181963A.

### (Liposome composition)

In connection with the route of administration, the liposome composition according to the embodiment of the present invention may also contain at least one of an isotonizing agent, a stabilizer, an antioxidant, or a pH adjusting agent which is pharmaceutically acceptable. That is, the liposome composition according to the embodiment of the present invention can be provided as a pharmaceutical composition.

The isotonizing agent is not particularly limited and examples thereof include inorganic salts such as sodium chloride, potassium chloride, sodium hydrogen phosphate, sodium dihydrogen phosphate, and potassium dihydrogen phosphate; polyols such as glycerol, mannitol, and sorbitol; and sugars such as glucose, fructose, lactose, and sucrose.

The stabilizer is not particularly limited and examples thereof include sugars such as glycerol, mannitol, sorbitol, lactose, and sucrose.

The antioxidant is not particularly limited and examples thereof include ascorbic acid, uric acid, tocopherol homologues (for example, vitamin E, four tocopherol isomers α, β, γ, and δ), cysteine, and ethylenediaminetetraacetic acid (EDTA). The stabilizer and the antioxidant may be each used alone or in a combination of two or more thereof.

Examples of the pH adjusting agent include sodium hydroxide, citric acid, acetic acid, triethanolamine, sodium hydrogen phosphate, sodium dihydrogen phosphate, and potassium dihydrogen phosphate.

The liposome composition according to the embodiment of the present invention may contain an organic solvent, collagen, polyvinyl alcohol, polyvinyl pyrrolidone, a carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, casein, gelatin, agar, diglycerin, propylene glycol, polyethylene glycol, vaseline, paraffin, stearyl alcohol, stearic acid, human serum albumin (HSA), mannitol, sorbitol, lactose, phosphate buffered saline (PBS), sodium chloride, sugars, a polymer degradable in vivo, a serum-free medium, each of which is pharmaceutically acceptable, or an additive which is acceptable as a medicinal additive.

The container in which the liposome composition according to the embodiment of the present invention is filled is not particularly limited, and it is preferably made out of a material having low oxygen permeability. Examples of the container include a plastic container, a glass container, and a bag formed from a laminated film which has, as a gas barrier layer, an aluminum foil, an aluminum vapor deposition film, an aluminum oxide vapor deposition film, a silicon oxide vapor deposition film, a polyvinyl alcohol, an ethylene-vinyl alcohol copolymer, a polyethylene terephthalate, a polyethylene naphthalate, a polyvinylidene chloride, or the like. The container can be shielded from light by employing, for example, a bag using a colored glass, an aluminum foil, an aluminum vapor deposition film, or the like, as necessary.

In the container in which the liposome composition is filled, in order to prevent oxidation due to oxygen existing in the space inside the container, it is preferable to replace a gas in the container space and a gas in the chemical liquid with an inert gas such as nitrogen. For example, an injection solution is bubbled with nitrogen, whereby the filling of the injection solution into a container can be carried out under a nitrogen atmosphere.

The route of administration of the liposome composition according to the embodiment of the present invention is preferably parenteral administration. Examples of the parenteral administration include intravenous injection such as intravenous drip infusion, intramuscular injection, intraperitoneal injection, subcutaneous injection, intraocular injection, and intrathecal injection. Examples of the administration method include administration with a syringe or drip infusion.

### (Dose rate in usage of liposome composition)

For the liposome composition according to the embodiment of the present invention, it is preferable that a dose per administration of the topotecan or salt thereof encompassed in the liposome is such that the dose rate thereof is 0.1 mg/m² body surface area to 10 mg/m² body surface area in terms of topotecan. It is more preferably 0.5 mg/m² body surface area to 5 mg/m² body surface area and still more preferably 1.0 mg/m² body surface area to 3.5 mg/m² body surface area.

It is preferable that the single administration of the liposome composition according to the embodiment of the present invention is repeatedly carried out a plurality of times every 1 week to 8 weeks. It is more preferable that the single administration is repeatedly carried out a plurality of times every 1 week to 6 weeks, it is still more preferable that the single administration is repeatedly carried out a plurality of times every 1 week to 4 weeks, and it is particularly preferable that the single administration is repeatedly carried out a plurality of times every two weeks.

The liposome composition according to the embodiment of the present invention is preferably administered by infusion over 5 minutes to 360 minutes in a single administration. 5 minutes to 240 minutes are more preferable, 10 minutes to 120 minutes are still more preferable, and 30 minutes to 120 minutes are particularly preferable.

The dose rate per administration of the topotecan or salt thereof contained in the liposome composition according to the embodiment of the present invention is, for example, about 0.1 mg/m² body surface area, about 0.5 mg/m² body surface area, about 1.0 mg/m² body surface area, about 1.5 mg/m² body surface area, about 2.0 mg/m² body surface area, about 2.5 mg/m² body surface area, about 2.6 mg/m² body surface area, about 3.0 mg/m² body surface area, about 3.5 mg/m² body surface area, about 4.0 mg/m² body surface area, about 4.5 mg/m² body surface area, about 5.0 mg/m² body surface area, about 5.5 mg/m² body surface area, about 6.0 mg/m² body surface area, about 6.5 mg/m² body surface area, about 7.0 mg/m² body surface area, about 7.5 mg/m² body surface area, about 8.0 mg/m² body surface area, about 8.5 mg/m² body surface area, about 9.0 mg/m² body surface area, about 9.5 mg/m² body surface area, or about 10 mg/m² body surface area in terms of topotecan. It is preferably about 0.5 mg/m², about 1.0 mg/m² body surface area, about 1.5 mg/m² body surface area, about 2.0 mg/m² body surface area, about 2.5 mg/m² body surface area, about 2.6 mg/m² body surface area, about 3.0 mg/m² body surface area, about 3.5 mg/m² body surface area, or about 5.0 mg/m² body surface area, more preferably about 1.0 mg/m² body surface area, about 1.5 mg/m² body surface area, about 2.0 mg/m² body surface area, about 2.5 mg/m² body surface area, about 2.6 mg/m² body surface area, about 3.0 mg/m² body surface area, or, about 3.5 mg/m² body surface area, and particularly preferably about 1.0 mg/m² body surface area, about 1.5 mg/m² body surface area, about 2.0 mg/m² a body surface area, about 2.5 mg/m² body surface area, about 2.6 mg/m² body surface area, or about 3.5 mg/m² body surface area.

### (DNA damage repair inhibitor)

The medicinal drug according to the embodiment of the present invention is used as a medicine that is used by combining a liposome composition encompassing topotecan salt thereof, where the liposome composition is used by being administered at the same time or sequentially together with a DNA damage repair inhibitor (DNA damage response inhibitor). The DNA damage repair inhibitor is one kind of anti-cancer agent. Cancer cells proliferate rapidly by actively undergoing cell division. Damage to a gene (DNA) occurs frequently during proliferation; however, in cancer, a biological molecule that repairs the damage may work strongly. An anti-cancer agent that causes cancer cells to die by inhibiting the function of repairing DNA damage is the DNA damage repair inhibitor.

Examples of the DNA damage repair inhibitor include a PARP inhibitor, an ATR inhibitor, an ATM inhibitor, a DNA-PK inhibitor, a CHK1/2 inhibitor, and a WEE1 inhibitor, as well as inhibitors that inhibit pathways in which these inhibitors are involved, where the inhibitors have a function of inhibiting a biological molecule that repairs DNA damage in cancer. Among the DNA damage repair inhibitors, a PARP inhibitor, an ATR inhibitor, an ATM inhibitor, a CHK1/2 inhibitor, a WEE1 inhibitor, and a DNA-PK inhibitor are preferable, an ATR inhibitor, an ATM inhibitor, and a PARP inhibitor are more preferable, and a PARP inhibitor is still more preferable in the medicinal drug according to the embodiment of the present invention.

Examples of the PARP inhibitor include olaparib, talazoparib, veliparib, niraparib, iniparib, and rucaparib, and olaparib is preferable.

Examples of the ATR inhibitor include ceralasertib, berzosertib, elimusertib, M1774, RP-3500, ATRN-119, ART0380, IMP9064, HRS2398, M4344, and BAY-1895344, and ceralasertib is preferable.

Examples of the ATM inhibitor include AZD0156, KU60019, AZD1390, M3541, and M4076, and M4076 is preferable.

Examples of the CHK1/2 inhibitor include prexasertib, MK-8776, AZD7762, LY2603618, GDC-0575, SRA-737, and ACR-368, and prexasertib is preferable.

Examples of the DNA-PK inhibitor include M3814, CC-115, and AZD7648, and M3814 is preferable.

Examples of the WEE1 inhibitor include ZN-c3, adavosertib, debio0123, IMP7068, and SY4835, and ZN-c3 is preferable.

In the present invention, one kind or a plurality of kinds of DNA damage repair inhibitors can be used. The DNA damage repair inhibitor can be obtained by purchasing a commercially available product.

The dose and the number of administrations of the DNA damage repair inhibitor according to the embodiment of the present invention may be appropriately set according to the kind of drug, the state of the patient, and the like. For example, a daily mass of the medicinal drug in terms of the mass of the active ingredient of the DNA damage repair inhibitor can be set in a range of about 0.1 mg to about 5,000 mg. In addition, examples of the route of administration of the DNA damage repair inhibitor include a route of oral administration and a route of parenteral administration (for example, injection, drip infusion, and administration into the rectal site), and the DNA damage repair inhibitor can be administered according to known clinical practice.

In a case where the DNA damage repair inhibitor according to the embodiment of the present invention is a PARP inhibitor, for example, a daily mass of the medicinal drug in terms of the mass of the active ingredient of the PARP inhibitor can be set in a range of about 0.1 mg to about 5,000 mg. It may be appropriately set according to the dose and the number of administrations described in the attached document for olaparib in a case where olaparib is used as the PARP inhibitor. In a case of olaparib, it is possible to orally administer olaparib in a range of 30 to 500 mg per administration.

In a case where the DNA damage repair inhibitor according to the embodiment of the present invention is an ATR inhibitor, for example, a daily mass of the medicinal drug in terms of the mass of the active ingredient of the ATR inhibitor can be set in a range of about 0.1 mg to about 5,000 mg. In a case where ceralasertib is used as the ATR inhibitor, the dose and the number of administrations may be appropriately set according to clinical records.

In a case where the DNA damage repair inhibitor according to the embodiment of the present invention is an ATM inhibitor, for example, a daily amount of the drug in terms of the amount of the active ingredient of the ATM inhibitor can be set in a range of about 0.1 mg to about 5,000 mg. In a case where M4076 is used as the ATM inhibitor, the dose and the number of administrations may be appropriately set according to clinical records.

In a case where the DNA damage repair inhibitor according to the embodiment of the present invention is a CHK1/2 inhibitor, for example, a daily mass of the medicinal drug in terms of the mass of the active ingredient of the CHK1/2 inhibitor can be set in a range of about 0.1 mg to about 5,000 mg. In a case where prexasertib is used as the CHK1/2 inhibitor, the dose and the number of administrations may be appropriately set according to clinical records.

In a case where the DNA damage repair inhibitor according to the embodiment of the present invention is a DNA-PK inhibitor, for example, a daily mass of the medicinal drug in terms of the mass of the active ingredient of the DNA-PK inhibitor can be set in a range of about 0.1 mg to about 5,000 mg. In a case where M3814 is used as the DNA-PK inhibitor, the dose and the number of administrations may be appropriately set according to clinical records.

In a case where the DNA damage repair inhibitor according to the embodiment of the present invention is a WEE1 inhibitor, for example, a daily mass of the medicinal drug in terms of the mass of the active ingredient of the WEE1 inhibitor can be set in a range of about 0.1 mg to about 5,000 mg. In a case where ZN-c3 is used as the WEE1 inhibitor, the dose and the number of administrations may be appropriately set according to clinical records.

The medicinal drug according to the embodiment of the present invention a medicine that is used by combining a liposome composition encompassing topotecan salt thereof and a DNA damage repair inhibitor, which are administered at the same time or sequentially, and it can be preferably used as an anti-cancer agent.

The cancer to which the medicinal drug according to the embodiment of the present invention is applied is preferably a solid cancer. Examples of the solid cancer include ovarian cancer, uterine cancer, lung cancer, Merkel cell cancer, skin cancer, breast cancer, malignant soft tissue tumor, neuroendocrine tumor, brain tumor, pharyngeal cancer, laryngeal cancer, thyroid cancer, esophageal cancer, gastric cancer, colon cancer, liver cancer, pancreatic cancer, gallbladder cancer, renal cancer, bladder cancer, prostate cancer, testis cancer, and bone tumor, where at least one selected from breast cancer, uterine cancer, ovarian cancer, lung cancer, Merkel cell cancer, neuroendocrine tumor, or brain tumor is preferable.

Examples of the ovarian cancer include serous ovarian cancer, endometrioid ovarian cancer, clear cell ovarian cancer, and mucous ovarian cancer, where serous ovarian cancer or endometrioid ovarian cancer is particularly preferable. In addition, the ovarian cancer may be ovarian cancer that is resistant to a platinum preparation (platina preparation).

Examples of the uterine cancer include cervical cancer, uterine body cancer, and uterine sarcoma, where cervical cancer or uterine sarcoma is particularly preferable.

Examples of the lung cancer include non-small cell lung cancer and small cell lung cancer, where small cell lung cancer is particularly preferable.

It has been found that in a case of being used by combining a liposome composition encompassing topotecan salt thereof and a DNA damage repair inhibitor, which are administered at the same time or sequentially, the medicinal drug according to the embodiment of the present invention has a strong anti-tumor effect (for example, an effect of suppressing tumor growth and the like) as compared with each of the single agents (the liposome composition encompassing topotecan salt thereof or the DNA damage repair inhibitor).

The action mechanism of the combined use of the liposome composition and the DNA damage repair inhibitor according to the embodiment of the present invention is presumed as follows; however, the action mechanism thereof is not limited thereto.

The topotecan or salt thereof encompassed in the liposome composition according to the embodiment of the present invention is a drug known as a topoisomerase I inhibitor. Topoisomerase I has a function of cleaving and rejoining one strand of the double helical structure of DNA during cell division. The inhibition of the topoisomerase I makes it possible to actively cause damage to DNA.

In addition, by allowing a liposome to encompass topotecan or a salt thereof, it is possible to reduce damage to the bone marrow without weakening the effect of damaging DNA in cancer cells.

The medicinal drug according to the embodiment of the present invention makes it also possible to administer the DNA damage repair inhibitor, after the administration of the liposome composition encompassing topotecan or a salt thereof. By administering a DNA damage repair inhibitor after the administration of the liposome composition according to the embodiment of the present invention, it is possible to maintain the therapeutic effect, prevent the recurrence of cancer, and prevent the progression of cancer, which makes it possible to expect the improvement of the QOL of the patient.

The medicinal drug according to the embodiment of the present invention makes it also possible to administer the liposome composition encompassing topotecan or a salt thereof, after the administration of the DNA damage repair inhibitor. By administering a DNA damage repair inhibitor after the administration of the liposome composition according to the embodiment of the present invention, it is possible to maintain the therapeutic effect, prevent the recurrence of cancer, and prevent the progression of cancer, which makes it possible to expect the improvement of the QOL of the patient.

In the medicinal drug according to the embodiment of the present invention, the ratio of the dose or blending amount of the liposome composition encompassing topotecan or a salt thereof, and the DNA damage repair inhibitor is not particularly limited as long as it is in a range in which the effect of enhancing the therapeutic effect with respect to cancer is exhibited.

For example, the dose of the topotecan or salt thereof in the liposome composition according to the embodiment of the present invention is set to about 0.0000001 to 100 times (mass ratio), preferably about 0.000001 to 10 times (mass ratio), more preferably about 0.00001 to 1 time (mass ratio), and still more preferably about 0.0001 to 0.1 times (mass ratio) the dose of the DNA damage repair inhibitor.

In a case where a PARP inhibitor is used as the DNA damage repair inhibitor, the dose of the liposome composition according to the embodiment of the present invention is set to about 0.0000001 to 100 times (mass ratio), preferably about 0.000001 to 10 times (mass ratio), more preferably about 0.00001 to 1 time (mass ratio), and still more preferably about 0.0001 to 0.1 times (mass ratio) the dose of the PARP inhibitor.

Among the above, in a case where olaparib is used as the PARP inhibitor, the dose of the liposome composition according to the embodiment of the present invention is set to about 0.0000001 to 100 times (mass ratio), preferably about 0.000001 to 10 times (mass ratio), more preferably about 0.00001 to 1 time (mass ratio), and still more preferably about 0.0001 to 0.1 times (mass ratio) the dose of olaparib.

In a case where an ATR inhibitor is used as the DNA damage repair inhibitor, the dose of the liposome composition according to the embodiment of the present invention is set to about 0.0000001 to 100 times (mass ratio), preferably about 0.000001 to 10 times (mass ratio), more preferably about 0.00001 to 1 time (mass ratio), and still more preferably about 0.0001 to 0.1 times (mass ratio) the dose of the ATR inhibitor.

In a case where an ATM inhibitor is used as the DNA damage repair inhibitor, the dose of the liposome composition according to the embodiment of the present invention is set to about 0.0000001 to 100 times (mass ratio), preferably about 0.000001 to 10 times (mass ratio), more preferably about 0.00001 to 1 time (mass ratio), and still more preferably about 0.0001 to 0.1 times (mass ratio) the dose of the ATM inhibitor.

In a case where a CHK1/2 inhibitor is used as the DNA damage repair inhibitor, the dose of the liposome composition according to the embodiment of the present invention is set to about 0.0000001 to 100 times (mass ratio), preferably about 0.000001 to 10 times (mass ratio), more preferably about 0.00001 to 1 time (mass ratio), and still more preferably about 0.0001 to 0.1 times (mass ratio) the dose of the CHK1/2 inhibitor.

In a case where a DNA-PK inhibitor is used as the DNA damage repair inhibitor, the dose of the liposome composition according to the embodiment of the present invention is set to about 0.0000001 to 100 times (mass ratio), preferably about 0.000001 to 10 times (mass ratio), more preferably about 0.00001 to 1 time (mass ratio), and still more preferably about 0.0001 to 0.1 times (mass ratio) the dose of the DNA-PK inhibitor.

In a case where a WEE1 inhibitor is used as the DNA damage repair inhibitor, the dose of the liposome composition according to the embodiment of the present invention is set to about 0.0000001 to 100 times (mass ratio), preferably about 0.000001 to 10 times (mass ratio), more preferably about 0.00001 to 1 time (mass ratio), and still more preferably about 0.0001 to 0.1 times (mass ratio) the dose of the WEE1 inhibitor.

The present invention will be described in more detail below according to Examples; however, the present invention is not limited to these Examples.

### Examples

Fully synthesized DHSM indicates dihydrosphingomyelin that is produced by chemical synthesis to contain 98% or more of the following compound having a long-chain alkyl group having 16 carbon atoms and a long-chain alkyl group having 18 carbon atoms.

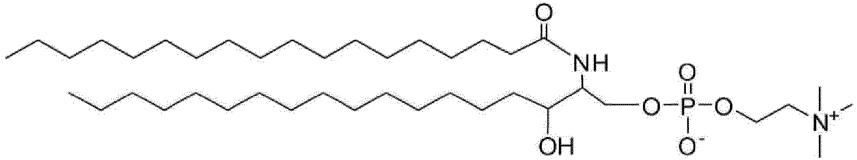

SUNBRIGHT DSPE-020CN (hereinafter referred to as DSPE-PEG, manufactured by NOF Corporation) was used as PEG phospholipid (denoted as PEG in the table).

Cholesterol HP (manufactured by Nippon Fine Chemical Co., Ltd.) was used as cholesterol (denoted as Chol in the table).

<Reference Example 1> Preparation of liposome composition encompassing topotecan salt thereof
(a) Preparation of oil phase
   For Example 1, 28.63 g of fully synthesized DHSM, 9.94 g of PEG phospholipid (SUNBRIGHT DSPE-020CN, manufactured by NOF Corporation, hereinafter referred to as DSPE-PEG), and 9.94 g of cholesterol were respectively weighed. The lipid was mixed with 711 mL of ethanol and dissolved at 68°C to prepare an oil phase.
(b1) Preparation of water phase 1
   58.0 g of ammonium sulfate was dissolved in 2,573.0 g of water to prepare a water phase 1.
(b2) Preparation of water phase 2
   11.6 g of ammonium sulfate was dissolved in 514.5 g of water to prepare a water phase 2.
(c) Formation of liposome particles by emulsification
   The water phase 1 prepared in (b1) was heated to 68°C, the whole of the oil phase prepared in (a) was added thereto, and then these phases were mixed with a precision emulsification disperser at a circumferential speed of 26 m/s for 60 minutes. Subsequently, the liquid temperature was adjusted to 56°C, and the resultant mixture was mixed for 30 minutes. Subsequently, after the water phase 2 at room temperature was added thereto, the resultant mixture was gently stirred with a paddle while heating at 65°C to evaporate the organic solvent and the water, and the heating and stirring were stopped at a time at which the liquid was concentrated until the osmotic pressure of the liquid reached 0.8 mS/cm, whereby the evaporation was stopped.
(e) Replacement of liposome outer water phase liquid by dialysis
   An aqueous solution of 3.15% by mass NaCl was used as a dialysis liquid. Using this dialysis liquid, the liquid obtained in (c) was subjected to cross-flow filtration at room temperature to remove ammonium sulfate present in the outer water phase to obtain liposomes in which the outer water phase was replaced with the dialysis liquid.
(f) Encompassment of topotecan in liposome particles by remote loading
   Water for injection was added to topotecan hydrochloride (manufactured by ScinoPharm Co., Ltd.) to a concentration of 5.9 mg/mL. Further, a 1 mol/L NaOH solution was added thereto while the liquid was stirred well, and the pH was adjusted to about 3 to dissolve topotecan. Liposomes were added to the resulting topotecan solution at a volume ratio of 1/1, followed by heating at 62°C for 30 minutes.
(g) Removal of outer water phase topotecan by dialysis
   A sucrose/histidine buffer consisting of 9.4% by mass sucrose and 10 mmol/L histidine was prepared as a dialysis liquid. Using this dialysis liquid, the liquid obtained in (f) was subjected to cross-flow filtration at room temperature to remove topotecan present in the outer water phase to obtain topotecan-containing liposomes in which the outer water phase was replaced with the dialysis liquid.

### [Measurement and evaluation of physical properties]

### <Average particle diameter>

In the present invention, the average particle diameter refers to a cumulant average particle diameter measured by a dynamic light scattering method. The cumulant average particle diameter, which was measured by a dynamic light scattering method with ELSZ-2000ZS (manufactured by Otsuka Electronics Co., Ltd.), was 114 nm.

### (Results of measurement of topotecan concentration)

The result obtained quantifying the topotecan concentration, which was obtained by subjecting a sample to measurement with a high performance liquid chromatography (HPLC) device 1290DAD (manufactured by Agilent Technologies, Inc.), was 104.8%. In addition, the amount of topotecan contained in the inside of the liposome was 99.9% with respect to the amount of topotecan contained in the entire liposome. Hereinafter, the details of the measurement method will be described.

### (Measurement of total amount of topotecan in liposome preparation)

The measurement was carried out according to a liquid chromatography/ultraviolet-visible absorbance detection method by using a sample solution obtained by dissolving the prepared liposome solution in methanol to which 0.1% trifluoroacetic acid was added, and a calibration curve standard solution prepared by diluting topotecan hydrochloride.

The concentration of topotecan in the inner water phase was calculated by subtracting the concentration of topotecan in the outer water phase from the concentration of topotecan in the entire water phase.

### (Preparation of sample for measuring topotecan concentration of outer water phase)

100 µL of a liposome dispersion liquid is measured out and then diluted by adding thereto 900 µL of 1 × PBS. 500 µL of this dispersion liquid was weighed out and loaded (7,400 × g, 5°C, 60 minutes) onto an ultrafiltration filter, and the filtrate was used as a sample for HPLC analysis. A universal refrigerated centrifuge 5922 manufactured by Kubota, Ltd. was used as the centrifuge.

### (Preparation of calibration curve standard solution)

About 15 mg of topotecan hydrochloride was weighed, the volume thereof was adjusted to 100 mL with methanol to which 0.1% of trifluoroacetic acid was added, and then the obtained solution was used as a stock solution of the standard solution. 2 mL of this stock solution was weighed out, and methanol to which 0.1% of trifluoroacetic acid was added was used to prepare a 50 mL calibration curve standard solution.

### (Measurement)

The measurement was carried out under the following conditions by liquid chromatography/UV-vis absorbance detection.
Measurement wavelength: 382 nm, column: ACQUITY UPLC CSH Fluoro-Phenyl, particle diameter: 1.7 µM, inner diameter: 2.1 mm, length: 10 cm (Waters Corporation)
Column temperature: constant temperature in vicinity of 40°C

The mobile phase was a mixed liquid of water/methanol/trifluoroacetic acid, and for the feeding of liquid in the mobile phase, the concentration gradient was controlled by changing the mixing ratio between the respective solvents.

Measurement was carried out under the conditions of a flow rate: 1.0 mL/minute, an injection volume: 12 µL, and an autosampler temperature: constant temperature in vicinity of 10°C.

### <Measurement of sulfate ion concentration>

The sample was measured with an ion chromatography apparatus 883 Basic IC plus (manufactured by Metrohm AG) to quantify the concentration of sulfate ions. The encompassed sulfate ions showed a content of 0.065 w/v%. It was found that the sulfate ions present in the outer water phase have a content of 0.0003 w/v% and most of the sulfate ions are encompassed. The amount of the encompassed sulfate ions with respect to the amount of topotecan encompassed in the entire water phase was 1.19 in terms of molar ratio.

The concentration of sulfate ions in the inner water phase was calculated by subtracting the concentration of sulfate ions in the outer water phase from the concentration of sulfate ions in the entire water phase. The concentration of sulfate ions in each water phase was measured as follows.

### (Concentration of sulfate ions in entire water phase)

100 µL of the liposome dispersion liquid was measured out, methanol was added thereto to accurately adjust the volume to 10 mL, and then mixing was carried out. 1,000 µL of the liquid was measured out, and the volume was accurately adjusted to 20 mL with a diluent solvent consisting of 3% glycerin, 12 mmol/L sodium hydrogen carbonate, and 0.6 mmol/L sodium carbonate. This solution was passed through a solid phase extraction cartridge oasis hlb plus light cartridge, 30 mg sorbent per cartridge (Waters Corporation), and the fraction was used for ion chromatographic analysis.

### (Concentration of sulfate ions in outer water phase)

100 µL of the liposome dispersion liquid was measured out, 900 µL of the diluent solvent was added thereto, and then mixing was carried out. 500 µL of the liquid was treated with an ultrafiltration filter Amicon Ultra-0.5 (molecular weight cut-off: 10k) (Merck Millipore), and the filtrate was used as a sample for ion chromatographic analysis.

Centrifugation conditions were 7,400 g, 5°C, and 30 minutes. A universal refrigerated centrifuge 5922 manufactured by Kubota, Ltd. was used as the centrifuge.

The liposome composition according to the embodiment of the present invention was produced based on the above-described Examples. It was confirmed that the molar ratio of sulfate ions in the inner water phase to the drug in the entire water phase was 0.36 or more.

### [Test Example 1]

The drug efficacy and the safety test in a pancreatic cancer cell line (Capan-1) model in the combined use of the liposome composition according to the embodiment of the present invention and olaparib as a PARP inhibitor

As a test substance, the topotecan-encompassing liposome composition prepared in Reference Example 1 (hereinafter, also referred to as "Lipo") was used. For the dilution of Lipo, a 5% glucose injection solution (manufactured by Otsuka Pharmaceutical Co., Ltd.) (hereinafter, also referred to as a Lipo diluent liquid) was used.

### <Example 1>

1 × 10⁷ Capan-1 cells, which are a human pancreatic cancer cell line, were transplanted into a female mouse Balb/cAJcl-nu/nu at the right subcutaneous tissue of the abdomen to form a subcutaneous tumor. Thereafter, 0.5 mg/kg of the Lipo, which had been diluted with a 5% glucose injection solution so that the concentration thereof was 0.05 mg/mL, was administered (i.v.) into the caudal vein. This administration was repeated once a week for 4 weeks. The day of the first administration was set as the 0th day. In addition, on the 0th day, 50 mg/kg of olaparib (MedChem Express), which had been diluted with 10% DMSO in 10% HP-βCYD-containing PBS so that the concentration thereof was 5 mg/mL, was administered intraperitoneally (i.p.). This administration was repeated once a day for 6 times, and after one day of drug withdrawal, the same administration cycle was repeated for a total of 4 weeks. The tumor suppressive effect of the administration according to the embodiment of the present invention was evaluated using the tumor volume as an indicator. In addition, a change in body weight was measured as an indicator of safety.

### <Comparative Example 1>

The procedure was carried out in the same manner as in Example 1, except that Lipo and olaparib were not administered but only the same amount of the diluent solvent was administered. That is, the drug was not administered.

### <Comparative Example 2>

The procedure was carried out in the same manner as in Example 1, except that Lipo was not administered but only the same amount of the diluent solvent was administered. That is, the administered drug was only olaparib.

### <Comparative Example 3>

The procedure was carried out in the same manner as in Example 1, except that olaparib was not administered but only the same amount of the diluent solvent was administered. That is, the administered drug was only Lipo.

### <Comparative Example 4>

Lipo was not administered, but 5 mg/kg of topotecan (manufactured by ScinoPharm Co., Ltd., Inc.), which had been diluted with a saline liquid (manufactured by Otsuka Pharmaceutical Co., Ltd.) to 0.5 mg/mL, was administered (i.v.) into the caudal vein. This administration was repeated once a week for 4 weeks. In addition, olaparib was not administered, but only the same amount of the diluent solvent was administered. Except for the above points, the procedure was carried out in the same manner as in Example 1. That is, the administered drug was topotecan which was not encompassed in the liposome.

### <Comparative Example 5>

Lipo was not administered, but 5 mg/kg of topotecan (manufactured by ScinoPharm Co., Ltd., Inc.), which had been diluted with a saline liquid (manufactured by Otsuka Pharmaceutical Co., Ltd.) to 0.5 mg/mL, was administered (i.v.) into the caudal vein. This administration was repeated once a week for 4 weeks. Except for these points, the procedure was carried out in the same manner as in Example 1. That is, the administered drug was topotecan and olaparib, which were not encompassed in the liposome.

### <Administration conditions in Example 1 and Comparative Examples 1 to 5>

The administration conditions for Example 1 and Comparative Examples 1 to 5 are summarized in order in Table 1.

**[Table 1]**

| | Administered drug | Lipo or topotecan | | Olaparib | |
|---|---|---|---|---|---|
| | | Dose mg/kg | Administration schedule | Dose mg/kg | Administration schedule |
| Example 1 | Lipo + Olaparib | 0.5 | Once every week × 4 weeks | 50 | (Once every day × 6 days, one day of drug withdrawal) × 4 weeks |
| Comparative Example 1 | Absent | - | - | - | - |
| Comparative Example 2 | Olaparib | - | - | 50 | (Once every day × 6 days, one day of drug withdrawal) × 4 weeks |
| Comparative Example 3 | Lipo | 0.5 | Once every week × 4 weeks | - | - |
| Comparative Example 4 | Topotecan | 5 | Once every week × 4 weeks | - | - |
| Comparative Example 5 | Topotecan + Olaparib | 5 | Once every week × 4 weeks | 50 | (Once every day × 6 days, one day of drug withdrawal) × 4 weeks |

### <Results of evaluation of drug efficacy and safety in Example 1 and Comparative Examples 1 to 5>

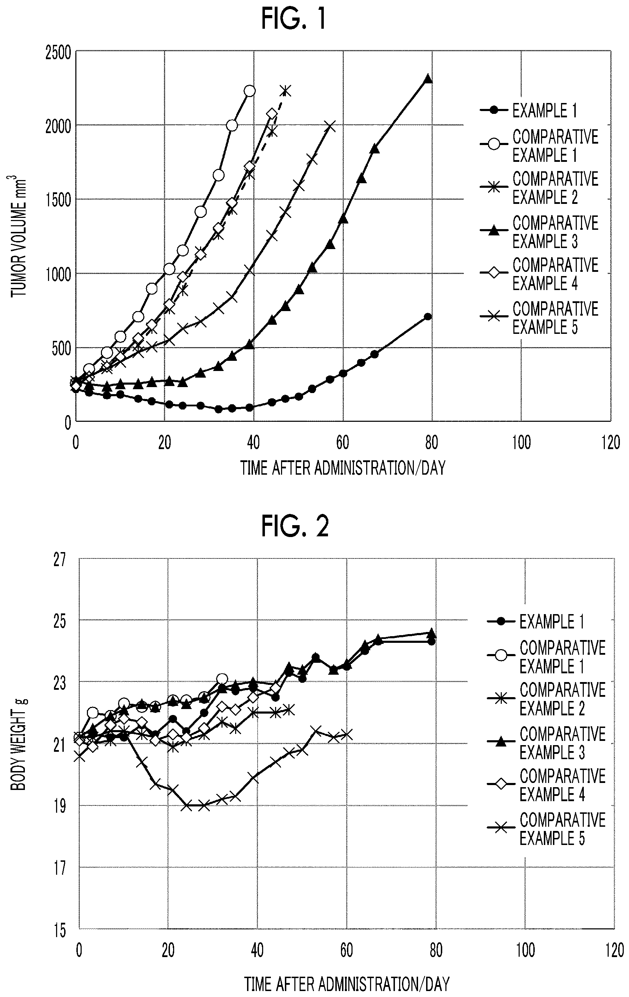

Table 2, Fig. 1, and Fig. 2 show the results of the evaluation of the drug efficacy (tumor diameter) and the safety (body weight) in Example 1 and Comparative Examples 1 to 5.

**[Table 2]**

| | Administered drug | Drug efficacy (tumor diameter: mm) 28 days after administration (Proportion to Comparative Example 1 is indicated in parentheses) | Safety (body weight: g) 28 days after administration (Proportion to Comparative Example 1 is indicated in parentheses) |
|---|---|---|---|
| Example 1 | Lipo + Olaparib | 105 (7%) | 105 (99%) |
| Comparative Example 1 | Absent | 1415 (100%) | 106 (100%) |
| Comparative Example 2 | Olaparib | 1143 (80%) | 103 (97%) |
| Comparative Example 3 | Lipo | 330 (23%) | 107 (101%) |
| Comparative Example 4 | Topotecan | 1126 (80%) | 102 (96%) |
| Comparative Example 5 | Topotecan + Olaparib | 673 (48%) | 90 (85%) |

Example 1 according to the embodiment of the present invention exhibited an extremely strong anti-tumor effect with a tumor diameter of 7% as compared with Comparative Example 1 in which no drug was administered 28 days after administration. On the other hand, it was found that there is almost no difference in body weight and the administration is carried out with excellent safety.

In addition, Example 1 exhibited a high anti-tumor effect even as compared with Comparative Example 3 in which only Lipo was used and Comparative Example 5 in which topotecan not encompassed in the liposome was used in combination with olaparib. Further, from the viewpoint of safety in which a change in body weight serves as an indicator, in Comparative Example 5 in which topotecan and olaparib were administered in combination, a significant decrease in body weight of 15% was observed as compared with Comparative Example 1 in which no drug was administered. This is considered to be due to the fact that strong toxicity was induced due to the combination of topotecan and olaparib. On the other hand, such enhancement of toxicity was not observed in the combined use of topotecan (Lipo) subjected to liposomization and olaparib. As described above, it is shown that the combined use of the topotecan subjected to liposomization and the DNA damage repair inhibitor such as olaparib, where the topotecan and the DNA damage repair inhibitor are according to the embodiment of the present invention, is a surprising invention that makes it possible to prevent an increase in toxicity and improve drug efficacy.

### [Test Example 2]

The tests for drug efficacy and safety in a pancreatic cancer cell line (Capan-1) model, in a case where the administration of the liposome composition according to the embodiment of the present invention is completed, and then olaparib as a PARP inhibitor, is sequentially administered

As a test substance, the liposome composition prepared in Reference Example 1 (hereinafter, also referred to as "Lipo") was used in the same manner as in Example 1. For the dilution of Lipo, a 5% glucose injection solution (manufactured by Otsuka Pharmaceutical Co., Ltd.) (hereinafter, also referred to as a Lipo diluent liquid) was used.

### <Example 2>

3 × 10⁶ Capan-1 cells, which are a human pancreatic cancer cell line, were transplanted into a female mouse Balb/cAJcl-nu/nu at the right subcutaneous tissue of the abdomen to form a subcutaneous tumor. Thereafter, 2 mg/kg of the Lipo, which had been diluted with a 5% glucose injection solution so that the concentration thereof was 0.05 mg/mL, was administered (i.v.) into the caudal vein. This administration was repeated once a week for 4 weeks. That is, in a case where the day of the first administration was set as the 0th day, the administration was carried out on the 0th day, the 7th day, the 14th day, and the 21st day. In addition, on the 24th day, which was 3 days after the completion of the administration of Lipo, 50 mg/kg of olaparib (MedChem Express), which had been diluted with 10% DMSO in 10% HP-βCYD-containing PBS so that the concentration thereof was 5 mg/mL, was administered intraperitoneally (i.p.). This administration was repeated once a day for 5 times, and after one day of drug withdrawal, the same administration cycle was repeated for a total of 4 weeks. The tumor suppressive effect of the administration according to the embodiment of the present invention was evaluated using the tumor volume as an indicator. In addition, a change in body weight was measured as an indicator of safety.

### <Example 3>

On the 0th day, together with the administration of Lipo, 50 mg/kg of olaparib (MedChem Express), which had been diluted with 10% DMSO in 10% HP-BCYD-containing PBS so that the concentration thereof was 5 mg/mL, was administered intraperitoneally (i.p.). This administration was repeated once a day for 5 times, and after one day of drug withdrawal, the same administration cycle was repeated for a total of 4 weeks. Except for the above points, the procedure was carried out in the same manner as in Example 2.

### <Comparative Example 6>

The procedure was carried out in the same manner as in Example 2, except that olaparib was not administered but only the same amount of the diluent solvent was administered. That is, the administered drug was only Lipo.

### <Administration conditions in Examples 2 and 3 and Comparative Example 6>

The administration conditions for Examples 2 and 3 and Comparative Example 6 are summarized in order in Table 3.

**[Table 3]**

| | Administered drug | Lipo or topotecan | | Olaparib | |
|---|---|---|---|---|---|
| | | Dose mg/kg | Administratio n schedule | Dose mg/kg | Administration schedule |
| Example 2 | Lipo + Olaparib | 2.0 | Once every week × 4 weeks | 50 | From 3 days after completion of administration of Lipo, (once every day × 6 days, one day of drug withdrawal) × 4 weeks |
| Example 3 | Lipo + Olaparib | 2.0 | Once every week × 4 weeks | 50 | At the same time on day of administration of Lipo, (once every day × 6 days, one day of drug withdrawal) × 4 weeks |
| Comparative Example 6 | Lipo | 2.0 | Once every week × 4 weeks | - | - |

### <Results of evaluation of drug efficacy and safety in Examples 2 and 3 and Comparative Example 6>

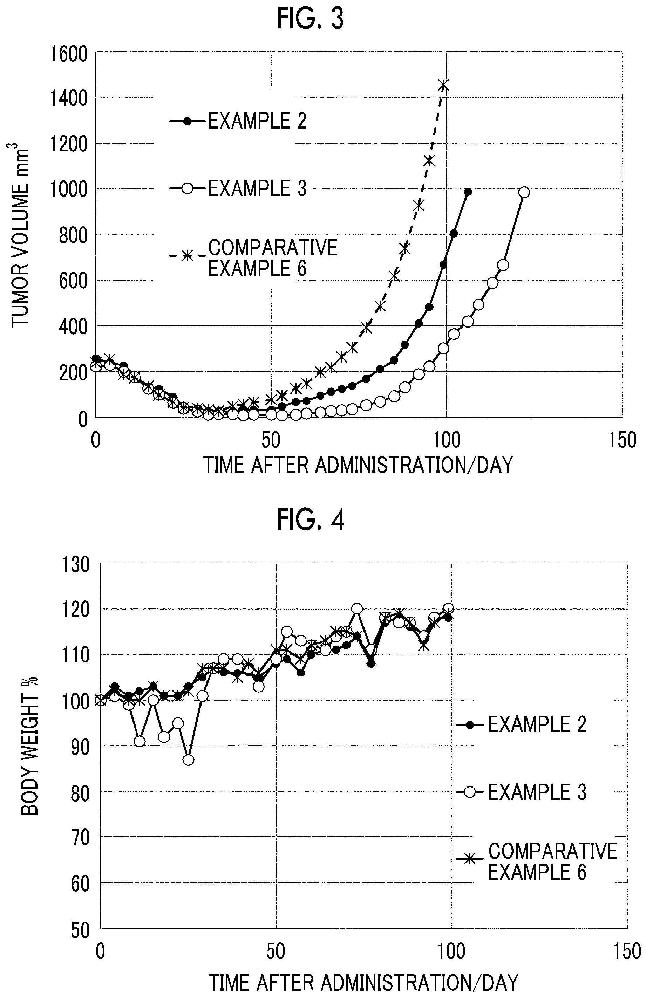

Table 4, Fig. 3, and Fig. 4 show the results of the evaluation of the drug efficacy (tumor diameter) and the safety (body weight) in Examples 2 and 3 and Comparative Example 6. The body weight indicates a change in a case where the body weight on the day of administration is set to 100%.

**[Table 4]**

| | Administered drug | Drug efficacy (tumor diameter: mm) 99 days after administration | Stability (% by body weight) 99 days after administration | Stability (% by body weight) 25 days after administration |
|---|---|---|---|---|
| Example 2 | Lipo + Olaparib | 668 | 118 | 103 |
| Example 3 | Lipo + Olaparib | 303 | 120 | 87 |
| Comparative Example 6 | Lipo | 1456 | 119 | 102 |

In Comparative Example 6 in which only Lipo was administered, the tumor grew large after a long period of time on the 99th day after 21 days from the completion of the administration of Lipo. On the other hand, in Examples 2 and 3 according to the embodiment of the present invention, the tumor diameter is maintained relatively small after a long period of time even on the 99th day after 21 days from the completion of the administration of Lipo, and thus a strong effect of suppressing tumor growth is observed. This is considered to be because the DNA damage repair inhibitor containing olaparib inhibits the repair of DNA damaged by topotecan, and thus the effect of suppressing tumor growth is maintained for a long period of time.

In Example 3 in which a period of administration of Lipo and a period of administration of olaparib are overlapped, the tumor diameter on the 99th day is small as compared with a case in Example 2, and thus a strong anti-tumor effect is exhibited; however, the body weight is relatively reduced significantly on the 25th day of administration. On the other hand, in Example 2, a large loss in body weight loss is not observed, and thus the safety is relatively high as compared with a case in Example 3. As a result, it was found that in a case where safety is considered to be important, the safety can be controlled by administering olaparib after the completion of the administration of Lipo as in Example 2.

### [Test Example 3]

The evaluation of the anti-tumor activity in the combined use of the liposome composition and the DNA damage repair inhibitor according to the embodiment of the present invention

In order to evaluate the synergistic effect obtained from the combined use of the liposome composition according to the embodiment of the present invention and various DNA damage repair inhibitors, an in vitro cell proliferation inhibition test was carried out under the conditions of the cell species, the number of cells seeded in the wells, and the culture conditions shown in Table 5. A Cell Counting Kit-8 (Dojindo Molecular Technologies. Inc.) was used for a living cell detection method, and the absorbance at 460 nm after 4 hours of reagent addition was measured with a microplate reader to obtain the IC₅₀ value.

**[Table 5]**

| Cell species | Capan-1 (pancreatic cancer cell line) | ES-2 (ovarian cancer cell line) | DMS114 (lung cancer cell line) |
|---|---|---|---|
| Number of cells | 6.0 × 10³ cells/well | 1.5 × 10³ cells/well | 2.0 × 10³ cells/well |
| Culture conditions | For 6 days after drug addition | For 3 days after drug addition | For 5 days after drug addition |
| | 37°C | 37°C | 37°C |
| | 5% CO₂ | 5% CO₂ | 5% CO₂ |

As various DNA damage repair inhibitors to be used in combination with the liposome composition, the following DNA damage repair inhibitors were used; ceralasertib (Cellex Biotechnology, Inc.) as an ATR inhibitor, prexasertib (Cellex Biotechnology, Inc.) as a CHK1/2 inhibitor, M3814 (Cellex Biotechnology, Inc.) as a DNA-PK inhibitor, M4076 (Cellex Biotechnology, Inc.) as an ATM inhibitor, ZN-c3 (Cellex Biotechnology, Inc.) as a WEE1 inhibitor, and olaparib (Cellex Biotechnology, Inc.) as a PARP inhibitor. Table 6 and Table 7 show the adding concentrations of the Lipo prepared in Reference Example 1 and the various DNA damage repair inhibitors for each cell specie.

**[Table 6]**

| With respect to Capan-1 (pancreatic cancer cell line) and DMS114 (lung cancer cell line) | | | | |
|---|---|---|---|---|
| Various DNA damage repair inhibitors (nmol/L) | | | | Liposome (nmol/L, *in terms of topotecan concentration) |
| Ceralasertib | Lipo | Prexasertib | Zn-C3 | Lipo |
| 0.0 | 0.0 | 0.0 | 0.0 | 0.0 |
| 4.6 | 0.1 | 0.05 | 1.4 | 0.1 |
| 13.7 | 0.4 | 0.1 | 4.1 | 0.4 |
| 41.2 | 1.2 | 0.4 | 12.3 | 1.2 |
| 123.5 | 3.7 | 1.2 | 37.0 | 3.7 |
| 370.4 | 11.1 | 3.7 | 111.1 | 11.1 |
| 1111.1 | 33.3 | 11.1 | 333.3 | 33.3 |
| 3333.3 | 100.0 | 33.3 | 1000.0 | 100.0 |
| 10000.0 | 300.0 | 100.0 | 3000.0 | 300.0 |

**[Table 7]**

| With respect to ES-2 (ovarian cancer cell line) | | | | |
|---|---|---|---|---|
| Various DNA damage repair inhibitors (nmol/L) | | | | Liposome (nmol/L, *in terms of topotecan concentration) |
| Ceralasertib, Zn-C3 | M4076, Olaparib | Prexasertib | M3814 | Lipo |
| 0.0 | 0.0 | 0.0 | 0.0 | 0.0 |
| 23.4 | 45.7 | 0.23 | 781.3 | 2.3 |
| 46.9 | 137.2 | 0.47 | 1562.5 | 4.7 |
| 93.8 | 411.5 | 0.93 | 3125.0 | 9.4 |
| 187.5 | 1234.6 | 1.9 | 6250.0 | 18.8 |
| 375.0 | 3703.7 | 3.8 | 12500.0 | 37.5 |
| 750.0 | 11111.1 | 7.5 | 25000.0 | 75.0 |
| 1500.0 | 33333.3 | 15.0 | 50000.0 | 150.0 |
| 3000.0 | 100000.0 | 30.0 | 100000.0 | 300.0 |

The synergistic effect obtained from the combined use was evaluated according to isobologram analysis, which is generally used to determine the combinational effect. First, the IC₅₀ value of Lipo alone and the IC₅₀ value of various DNA damage repair inhibitors alone were determined. Next, various DNA damage repair inhibitors were used in combination at each of the set concentration points of Lipo, and the IC₅₀ value of the DNA damage repair inhibitors was determined. A value obtained by dividing the IC₅₀ value of the DNA damage repair inhibitors by the IC₅₀ value of Lipo alone was denoted as "Lipo FIC", and a value obtained by dividing this value by the IC₅₀ value of the various DNA damage repair inhibitors was denoted as "various DNA damage repair inhibitor FIC", and then these two values were recorded as one set of values. As a part thereof, Table 8 shows each FIC in a case where Lipo and ceralasertib as an ATR inhibitor are used in combination in Capan-1.

In addition, Lipo was used in combination at each of the concentration points of the various DNA damage repair inhibitors which were set in the same manner, and the IC₅₀ value of the Lipo was determined. A value obtained by dividing this value by the IC₅₀ value of Lipo alone was denoted as "Lipo FIC", and a value obtained by dividing this value by the IC₅₀ value of the various DNA damage repair inhibitors was denoted as "various DNA damage repair inhibitor FIC", and then these two values were recorded as one set of values. As a part thereof, Table 9 shows each FIC in a case where Lipo and ceralasertib as an ATR inhibitor are used in combination in Capan-1.

**[Table 8]**

| Lipo (nmol/L) | IC₅₀ value (nmol/L) of DNA damage repair inhibitor (Ceralasertib) at each of concentration points of Lipo | DNA damage repair inhibitor (Ceralasertib) FIC | Lipo FIC |
|---|---|---|---|
| 11.1 | 14.9 | 0.02 | 1.3 |
| 3.7 | 181.3 | 0.2 | 0.4 |
| 1.23 | 798.5 | 0.9 | 0.1 |
| 0.41 | 809.4 | 0.9 | 0.05 |
| 0.14 | 634.4 | 0.7 | 0.02 |
| 0.0 | 893.0 | 1.0 | 0.0 |

**Table 9**

| DNA damage repair inhibitor (Ceralasertib) (nmol/L) | IC₅₀ value (nmol/L) of Lipo at each of concentration points of DNA damage repair inhibitor (Ceralasertib) | DNA damage repair inhibitor (Ceralasertib) FIC | Lipo FIC |
|---|---|---|---|
| 370 | 2.8 | 0.4 | 0.3 |
| 123 | 7.3 | 0.1 | 0.8 |
| 41.2 | 8.7 | 0.05 | 1.0 |
| 13.7 | 9.0 | 0.02 | 1.0 |
| 4.57 | 9.4 | 0.01 | 1.1 |
| 0.0 | 8.7 | 0.0 | 1.0 |

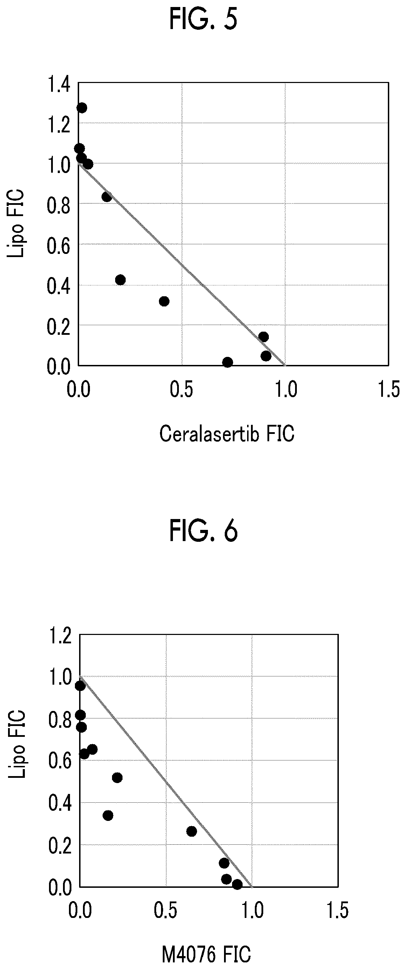

As shown in Table 8 and Table 9, an isobologram was obtained by plotting each set of these values on the graph of various DNA damage repair inhibitors FIC v.s. Lipo FIC. In the isobologram, a case of being plotted on a left side of a straight line obtained by connecting the points where the FIC on each axis is 1, indicates an "synergistic effect", which is regarded as a preferable effect of combined use. A case of being plotted on the straight line indicates an "additive effect". A case of being plotted to the right indicates an "antagonistic effect", which is regarded as an inappropriate effect of combined use. Fig. 5 to Fig. 21 show the results.

### [Results of examination of Capan-1]

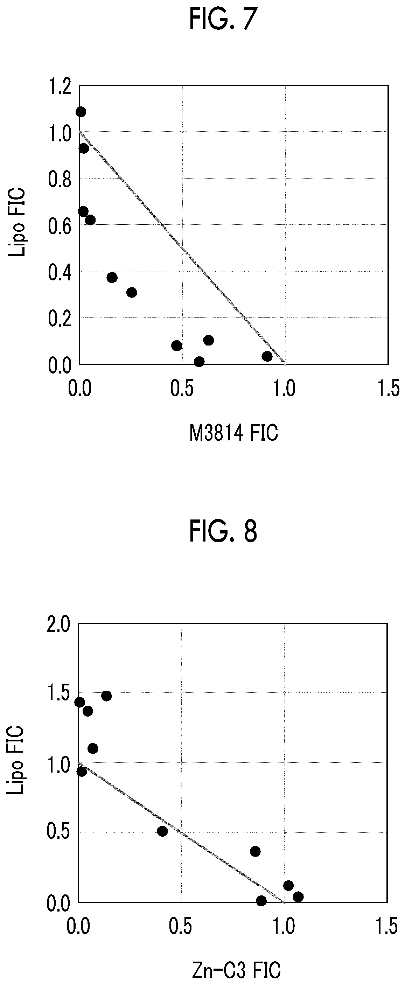

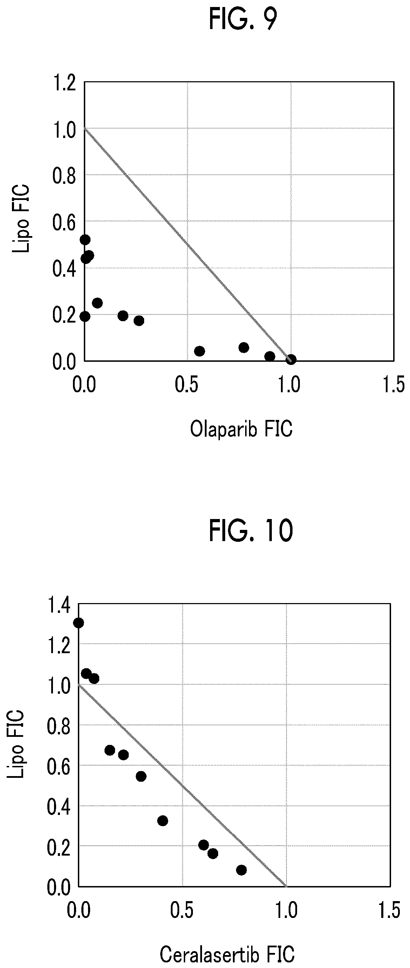

Fig. 5 shows the test results of ceralasertib, Fig. 6 shows the test results of M4076, Fig. 7 shows the test results of M3814, Fig. 8 shows the test results of ZN-c3, and Fig. 9 shows the test results of olaparib. As shown in Fig. 5, Fig. 7, and Fig. 9, a clear synergistic effect was observed in a case of ceralasertib, M3814, and olaparib. In addition, as shown in Fig. 6 and Fig. 8, an additive effect was observed in a case of M4076 and ZN-c3.

### [Results of examination of ES-2]

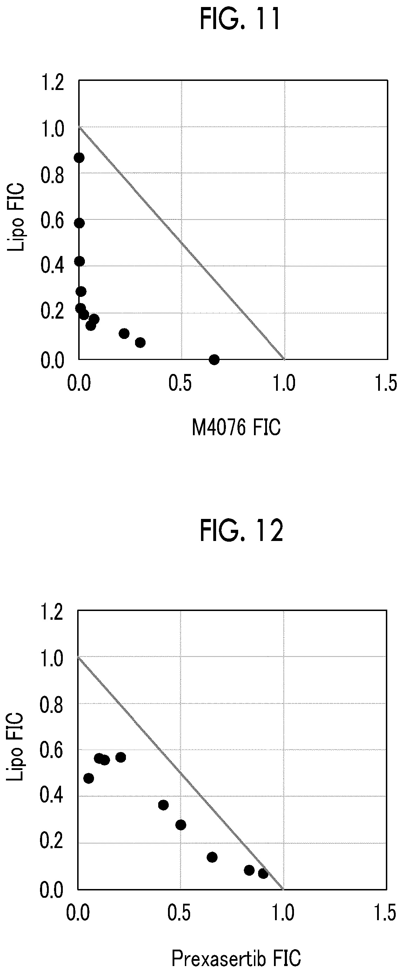

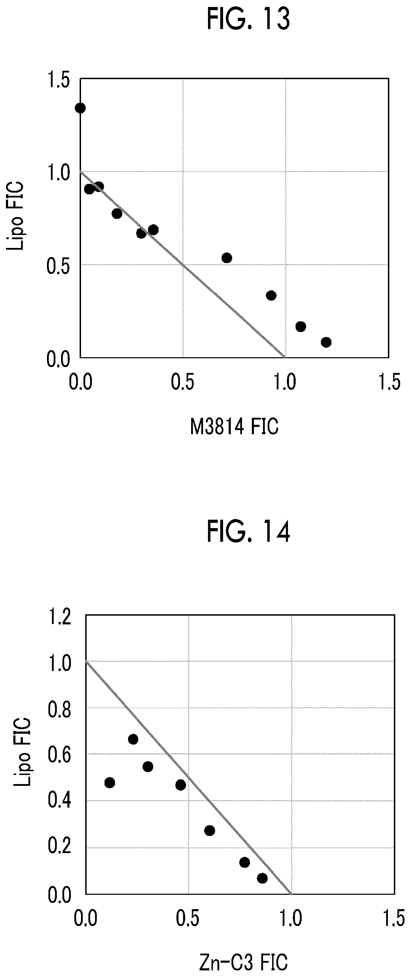

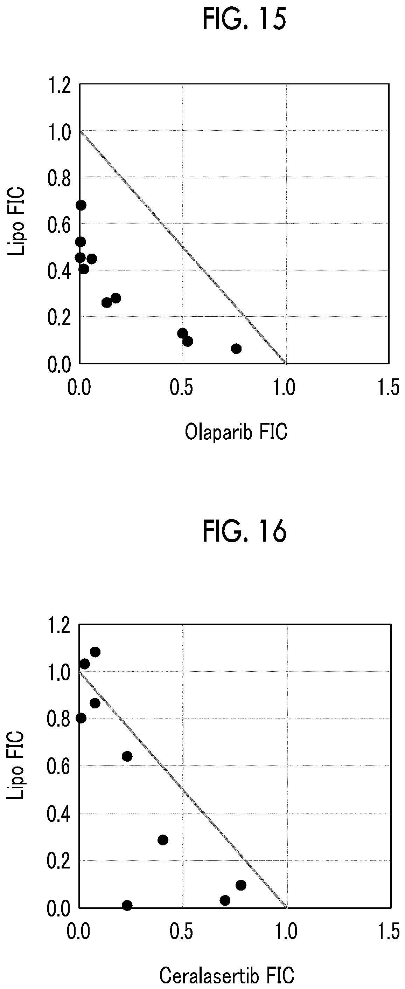

Fig. 10 shows the test results of ceralasertib, Fig. 11 shows the test results of M4076, Fig. 12 shows the test results of prexasertib, Fig. 13 shows the test results of M3814, Fig. 14 shows the test results of ZN-c3, and Fig. 15 shows the test results of olaparib. As shown in Fig. 10, Fig. 11, Fig. 12, Fig. 14, and Fig. 15, a clear synergistic effect was observed in a case of ceralasertib, prexasertib, M4076, ZN-c3, and olaparib. In addition, as shown in Fig. 13, an additive effect was observed in a case of M3814.

### [Results of examination of DMS 114]

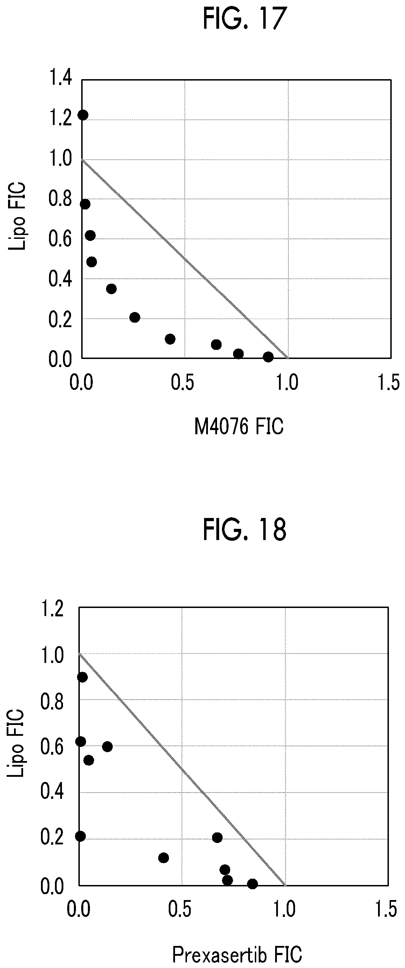

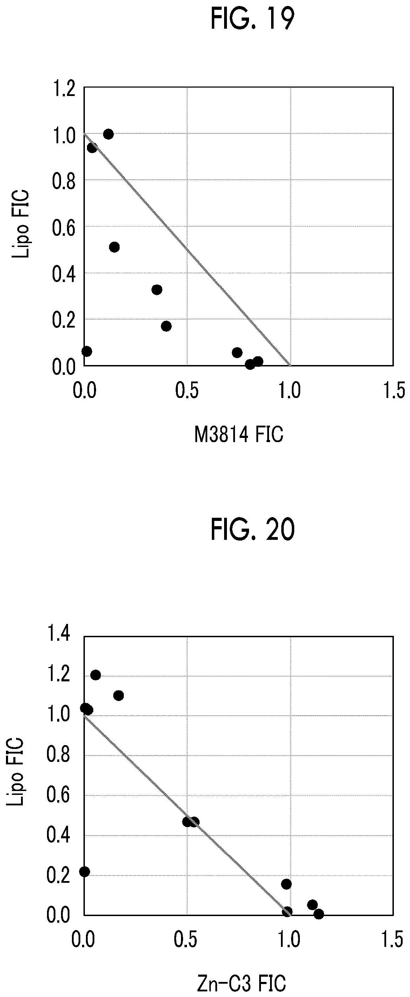

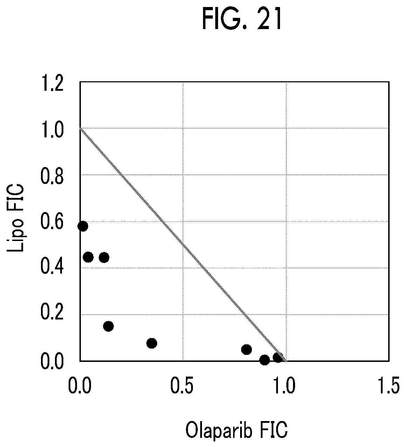

Fig. 16 shows the test results of ceralasertib, Fig. 17 shows the test results of M4076, Fig. 18 shows the test results of prexasertib, Fig. 19 shows the test results of M3814, Fig. 20 shows the test results of ZN-c3, and Fig. 21 shows the test results of olaparib. As shown in Fig. 16, Fig. 17, Fig. 18, Fig. 19, and Fig. 21, a clear synergistic effect was observed in a case of ceralasertib, prexasertib, M3814, M4076, and olaparib. In addition, as shown in Fig. 20, an additive effect was observed in a case of ZN-c3.

### [Test example 4]

The test for the combinational effect of the liposome composition and the DNA damage repair inhibitor according to the embodiment of the present invention in the cancer patient

As a test substance, a liquid medicinal preparation (hereinafter, referred to as an A preparation) containing topotecan-encompassing liposomes having the following composition is prepared with reference to WO2018/181963A.

| | |
|---|---|
| Topotecan hydrochloride | 3.0 mg/mL (6.5 mmol) |
| Fully synthesized DHSM | 10.4 mg/mL |
| DSPE-MPEG2000 | 3.6 mg/mL |
| Cholesterol | 3.6 mg/mL |
| Ammonium sulfate | 0.9 mg/mL (6.9 mmol) |
| Sucrose | appropriate amount |
| L-histidine | appropriate amount |
| Sodium chloride | appropriate amount |
| Water (solvent) | appropriate amount |

### <Physical property value of A preparation>

The pH of the A preparation was 7.4, and the particle diameter of the liposome contained in the A preparation was 91 nm in terms of the cumulant average particle diameter. In addition, the molar ratio of the sulfate ions contained in the inner water phase to the topotecan hydrochloride is 1.05.

### <Determination of administration and therapeutic effect>

The A preparation is administered to a cancer patient once every 1 week to 8 weeks such that the single administration dose of topotecan is 0.1 mg/m² body surface area to 10 mg/m² body surface area. In addition, the DNA damage repair inhibitor is administered according to known clinical practice under the guidance of a doctor. For example, in a case where the DNA damage repair inhibitor is olaparib, the DNA damage repair inhibitor is orally administered in a range of 30 to 500 mg per administration.

The effect of treatment can be determined according to the following standards.

The subjects to be subjected to evaluation are examined by diagnostic imaging using magnetic resonance imaging (MRI), and the evaluation is made according to the following standards.

Complete response (CR): A state in which the tumor has completely disappeared.

Partial response (PR): A state in which the sum of the sizes of the tumor has reduced by 30% or more.

Stable disease (SD): A state in which the size of the tumor has not changed.

Progressive disease (PD): A state in which the sum of the sizes of the tumor size has been increased by 20% or more and the absolute value thereof has been increased by 5 mm or more or a state in which a new lesion has appeared.

An excellent anti-tumor effect is obtained from the medicinal drug according to the embodiment of the present invention. Specifically, it is a therapeutic effect with respect to cancer without enhancing side effects on the normal organs, the bone marrow, and the like (such an effect of reducing the volume of cancer or an effect of not changing the size of a tumor). In addition, it is also possible to expect effects such as prevention of recurrence of cancer and prevention of progression of cancer due to the maintenance of the therapeutic effect.

From these results, the medicinal drug according to the embodiment of the present invention is expected to prolong the progression-free survival time and the overall survival time of patients, and it has a very useful effect from the viewpoint of improving the QOL of patients.

The medicinal drug according to the embodiment of the present invention exhibits an excellent therapeutic effect with respect to cancer and thus is useful.

## Claims

1. A medicinal drug comprising in combination:
(A) a liposome composition that contains a liposome having an inner water phase and contains an aqueous solution which is an outer water phase and disperses the liposome, where the liposome encompasses topotecan or a salt thereof, and a lipid constituting the liposome includes dihydrosphingomyelin; and
(B) a DNA damage repair inhibitor
wherein the liposome composition and the DNA damage repair inhibitor are administered at the same time or sequentially.

2. The medicinal drug according to claim 1,
wherein (A) the liposome composition is administered, and then (B) the DNA damage repair inhibitor is administered.

3. The medicinal drug according to claim 1,
wherein (B) the DNA damage repair inhibitor is administered, and then (A) the liposome composition is administered.

4. The medicinal drug according to any one of claims 1 to 3,
wherein the DNA damage repair inhibitor is at least one selected from a PARP inhibitor, an ATR inhibitor, an ATM inhibitor, a CHK1/2 inhibitor, a WEE1 inhibitor, a DNA-PK inhibitor, or inhibitors that inhibit pathways in which these inhibitors are involved.

5. The medicinal drug according to any one of claims 1 to 3,
wherein the lipid constituting the liposome further includes a lipid modified with cholesterol and polyethylene glycol.

6. The medicinal drug according to any one of claims 1 to 3,
wherein a formulation ratio of a lipid modified with polyethylene glycol to an entire lipid constituting the liposome is 2% by mole to 10% by mole.

7. The medicinal drug according to any one of claims 1 to 3,
wherein a lipid modified with polyethylene glycol is diacylphosphatidylethanolamine modified with polyethylene glycol or methoxypolyethylene glycol.

8. The medicinal drug according to any one of claims 1 to 3,
wherein a formulation ratio of cholesterol to an entire lipid constituting the liposome is 35% to 43% by mole.

9. The medicinal drug according to any one of claims 1 to 3,
wherein the inner water phase of the liposome contains an ammonium salt.

10. The medicinal drug according to any one of claims 1 to 3,
wherein the DNA damage repair inhibitor is a PARP inhibitor.

11. The medicinal drug according to any one of claims 1 to 3,
wherein a dose per administration of the topotecan or salt thereof encompassed in (A) the liposome composition is such that a dose rate is 0.1 mg/m² body surface area to 10 mg/m² body surface area in terms of topotecan.

12. The medicinal drug according to any one of claims 1 to 3,
wherein (A) the liposome composition is administered once every 1 week to 8 weeks.
